# EUROPEAN PATENT APPLICATION

(11) **EP 3 851 163 A1**
(43) Date of publication of application: **21.07.2021**
(21) Application number: 19860576.8
(22) Date of filing: 18.02.2019
(51) Int. Cl.: A61N 7/00

(54) **MULTI-CHANNEL ULTRASOUND STIMULATOR**

(30) Priority: 14.09.2018 KR 20180110361
(71) Applicant: Daegu-Gyeongbuk Medical Innovation Foundation, Daegu 41061 (KR)
(72) Inventor: PARK, Ju Young, Gyeongsan-si, Gyeongsangbuk-do 38538 (KR); JIN, Chang Zhu, Daegu 41066 (KR); HUH, Hyung Kyu, Daegu 41061 (KR)
(74) Representative: EP&C
(86) International application number: PCT/KR2019/001910
(87) International publication number: WO 2020/054929

(57) **Abstract**

Disclosed is an ultrasound stimulator used to increase a treatment effect of an anticancer drug by applying ultrasonic waves to the body of a user. The ultrasound stimulator includes a housing with a concavely curved inner surface and a plurality of channels disposed on the inner surface and spaced from each other at regular intervals. Each of the channels can emit ultrasonic waves independently of each other, and the channels form a wide ultrasound field range. The channels are arranged in a zigzag pattern. The channels can generate an ultrasound field to uniformly stimulate breast tissue of a patient due to the arrangement thereof on the concavely curved surface. In addition, a rotator rotates the housing about a rotary shaft to increase an application range of ultrasonic waves and to prevent local concentration of application of ultrasonic waves. A controller operates the channels according to a predetermined operation sequence according to the intensity of ultrasonic waves of each channel, thereby preventing excessive stimulation and enabling uniform stimulation.

## Description

### Technical Field

The present invention relates to a multi-channel ultrasonic stimulator and, more particularly, to a multi-channel ultrasonic stimulator used to increase the effect of treatment of cancer such as a breast cancer by enhancing the efficiency of ultrasonic stimulation.

### Background Art

Treatments of cancers such as a breast cancer are divided into surgical treatment and non-surgical anticancer treatment. Chemotherapy is one type of non-surgical anticancer treatment.

The non-surgical cancer treatment is very important in preventing recurrence and metastasis of cancer cells. Recently, there have been intensive studies how to reduce pain and increase the therapeutic effect of anticancer drugs. To this end, ultrasonic waves are used to generate mechanical vibration and heat in the body. This actively stimulates muscle fibers to reduce pain and increases the blood flow and nerve transmission rates to increase the therapeutic effect of anticancer drugs.

Korean Patent Application Publication No. 10-2011-0084802 (Patent Document 1) discloses an ultrasonic probe used to generate high-intensity focused ultrasound (HIFU). According to Patent Document 1, a plurality of probes is disposed on an upper portion of a housing to form a spherical surface, so that a wide ultrasonic sound field can be formed depending on the shape of the upper portion of the housing.

However, the technology disclosed in Patent Document 1 has problems. For example, it is difficult to expect even stimulation across the user's body because ultrasonic waves are emitted from a plurality of probe units spaced apart from each other. In addition, when the ultrasonic intensity is strong, excessive stimulation is applied to the inside of the user's body tissue.

Korean Patent Application Publication No. 10-2015-0003450 (Patent Document 2) discloses an ultrasound scanning aid for breast cancer diagnosis. The scanning aid has a structure in which the inside of a fixing container attached to the breast is irradiated with ultrasonic waves.

However, the device disclosed in Patent Document 2 has problems in that it is inconvenient to carry, it is difficult to uniformly stimulate a user's body because ultrasound waves are emitted from a specific location, and it is impossible to adaptively respond according to the intensity of the ultrasound.

### [Document of Related Art]

### [Patent Document]

[Patent Document 1] Korean Patent Application Publication No. 10-2011-0084802
[Patent Document 2] Korean Patent Application Publication No. 10-2015-0003450

### Disclosure

### Technical Problem

The present invention has been made to address the problems occurring in the related arts described above, and an objective of the present invention is to provide a multi-channel ultrasonic stimulator that is easy to carry and can reduce an excessive load on a user's body by adaptively adjusting an ultrasonic irradiation sequence according to the intensity of ultrasonic waves and a multi-channel ultrasonic stimulator capable of evenly stimulating the entire user's body even though ultrasonic waves are emitted through multiple channels.

### Technical Solution

In order to achieve the objective of the present invention, there is provided a multi-channel ultrasonic stimulator including a housing having a concavely curved inner surface and a plurality of channels disposed on the inner surface of the housing and spaced a predetermined distance from each other to independently emitting ultrasonic waves to form a wide ultrasonic field, in which the channels are arranged in a zigzag pattern.

The channels may be divided into a first group spaced apart from the center of the housing by a first distance and a second group spaced apart from the center of the housing by a second distance, the channels in each of the first and second groups are arranged at regular intervals, the second distance is longer than the first distance, and each of the channels in the second group is spaced from each of the adjacent channels in the first group.

A charging unit connected to each of the plurality of channels and serving to charge the channels with external electric power may be further included, and the channels may convert the electric power supplied from the charging unit into ultrasonic waves and emit the ultrasonic waves to the outside.

A rotator connected to the housing and configured to rotate the housing about a central axis may be further provided.

A controller for controlling the intensity of ultrasonic waves emitted from the plurality of channels may be further included. The controller may control the intensity of ultrasonic waves by controlling each of the channels to emit ultrasound waves according to a predetermined sequence.

The predetermined sequence may be configured such that each of the channels disposed along a circumferential direction of the housing sequentially emits ultrasonic waves one after another, and the intervals of the channels that sequentially emit ultrasonic waves increase with output values of the ultrasonic waves emitted from the channels.

### Advantageous Effects

According to the present invention, since the rotator rotates the housing, the positions of the channels are changed. Therefore, the stimulus applied to the user's body can be uniform.

In addition, since the emission order of the channels changes depending on the irradiation intensity of the ultrasonic waves according to the sequence that is set by the controller, it is possible to prevent the user's body from being excessively stimulated.

In addition, it is possible to increase the therapeutic effect of anticancer drugs by performing body stimulation with the ultrasonic stimulator according to the present invention before administering or injecting the anticancer drugs.

### Description of Drawings

FIG. 1 is a perspective view illustrating a multi-channel ultrasonic stimulator according to one embodiment of the present invention.
FIGS. 2 and 3 are conceptual diagrams illustrating multiple channels disposed on an inner surface of a housing according to one embodiment of the present invention.
FIG. 4 is a perspective view illustrating a multi-channel ultrasonic stimulator according to one embodiment of the present invention.
FIGS. 5 to 7 are conceptual diagrams illustrating an ultrasonic irradiation range when the housing is rotated.
FIG. 8 is a block diagram illustrating a controller according to one embodiment of the present invention.
FIGS. 9 and 10 are conceptual diagrams illustrating a sequence of operations performed by the controller according to one embodiment of the present invention.

### Best Mode

In the following description of embodiments of the present invention, when detailed descriptions of well-known configurations or functions is determined as interfering with understanding of the embodiments of the present invention, they are not described in detail.

Embodiments according to the concept of the present invention may be diversely modified and may have various shapes. Therefore, examples of the embodiments are illustrated in the accompanying drawings and will be described in detail herein with reference to the accompanying drawings.

The terminology used herein is for the purpose of describing particular embodiments only and is not intended to limit the claimed invention. As used herein, the singular forms "a", "an", and "the" are intended to include the plural forms as well, unless the context clearly indicates otherwise. It will be further understood that the terms "comprises" and/or "comprising", or "includes" and/or "including", or "has" and/or "having" when used in this specification, specify the presence of stated features, regions, integers, steps, operations, elements and/or components, but do not preclude the presence or addition of one or more other features, regions, integers, steps, operations, elements, components and/or groups thereof.

Hereinafter, the present invention will be described in detail.

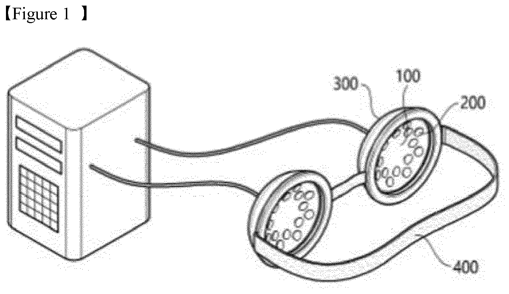

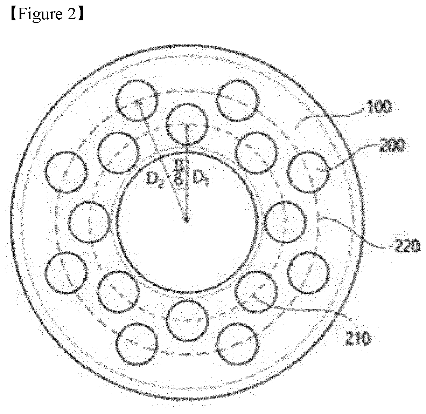

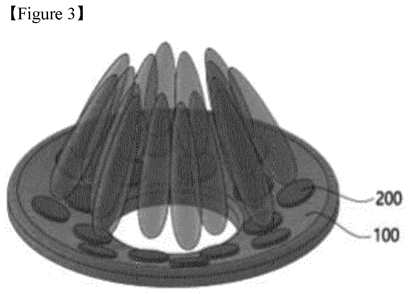

FIG. 1 is a perspective view illustrating a multi-channel ultrasonic stimulator according to one embodiment of the present invention, and FIGS. 2 and 3 are conceptual diagrams illustrating a plurality of channels 200 disposed inside a housing 100 according to one embodiment of the present invention. FIG. 2 is a conceptual diagram illustrating an arrangement example of the channels 200, and FIG. 3 shows a direction in which ultrasonic waves are emitted from the channel 200.

Referring to FIGS. 1 to 3, the multi-channel ultrasonic stimulator according to one embodiment of the present invention includes a housing 100 having a concavely curved surface and a plurality of channels 200 spaced a predetermined distance from each other on the concavely curved inner surface of the housing 100. Each of the channels 200 can independently emit ultrasonic waves to form an extensive ultrasonic sound field. The channels 200 are arranged in a zigzag pattern on the inner surface of the housing 100. In the present invention, it is preferable that the channels 200 emit ultrasonic waves in a sequential order rather than simultaneously emitting. With this operation, it is possible to prevent excessive stimulation of a user's body and to enable even ultrasonic stimulation through the entire surface to which ultrasonic waves are applied.

The housing 100 of the present invention has a concave shape, and a user's body part to be treated may be positioned in the cavity defined by the concave shape. The degree of concaveness of the housing 100 may vary depending on the shape of a user's body part to be treated, for example, the shape of a breast.

In one embodiment, a fixing portion 331 to come into contact with a user's body part is provided on an outer surface of the housing 100. The fixing portion 331 is made of a soft cushioning material such as silicone or rubber. The fixing portion 331 is not necessarily directly connected to or fixed to the housing 100. The fixing portion 331 may be separated from the housing 100 and will come into contact with a user's body part when used. The user may place the fixing portion 331 on the user's chest and put the user's breast into the housing 100 to stimulate the breast with ultrasonic waves emitted from the channels 200. In this case, the housing 100 is positioned a predetermined distance apart from the user's breast.

The channels 200 are arranged in a predetermined pattern on the inner surface of the housing 100 to emit ultrasonic waves. The channels 200 are spaced apart from each other and can emit ultrasonic waves independently of each other. The channels 200 convert electric power that is externally supplied into ultrasonic waves and emit the ultrasonic waves, thereby providing vibration energy or thermal energy to a user's body part. An example of the channel is a piezoelectric element. The channel 200 including a piezoelectric element converts an input voltage into mechanical energy using a piezoelectric effect and applies ultrasonic waves having a specific frequency. In addition, the channel 200 optionally includes a voltage converter that converts an input voltage and a transducer that forms an ultrasonic wave on the basis of the converted voltage. The channel 200 further optionally includes a signal amplifier that adjusts the converted voltage or the intensity of the ultrasonic wave.

As illustrated in FIG. 2, in the present invention, the plurality of channels 200 is arranged in a predetermined pattern in the housing 100. In one embodiment, the plurality of channels 200 is divided into a first channel 210 spaced apart by a first distance D1 from the center of the housing and a second group 220 spaced apart by a second distance D2 from the center of the housing 100. In each of the first and second groups 210 and 220, the channels are arranged at regular intervals.

Specifically, the second distance D2 is longer than the first distance D1. That is, the channels 200 constituting the second group 220 are closer to the center of the housing 100 than the channels 200 constituting the first group 210. In addition, the arrangement interval between each of the channels 200 constituting the first group 210 is shorter than the arrangement interval between each of the channels 200 constituting the second group 220. Each of the channels included in the second group 220 is equally distanced from each of the adjacent channels included in the first group 210. In other words, the plurality of channels 200 is radially arranged in a zigzag pattern. The difference between the second distance D2 and the first distance D1 is preferably smaller than the diameter of the channel 200 in terms of securing an application range of ultrasonic waves. In one embodiment, 16 channels are provided in total. In this case, the first group is composed of eight channels and the second group is composed of eight channels 200. The number of channels 200 may vary.

In addition, as illustrated in FIG. 3, the ultrasonic waves emitted from the channels 200 included in the first group 210 and the ultrasonic waves emitted from the channels 200 included in the second group 220 are concentrated on a specific region due to the concave shape of the housing 100. The specific region on which the ultrasonic waves are concentrated may be present on a central axis of the housing 100. However, even in this case, it is preferable that all the channels 200 simultaneously emit the ultrasonic waves to the concentrated point.

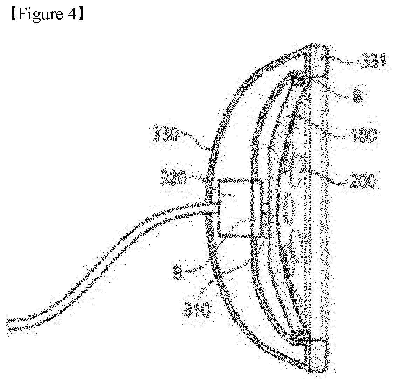

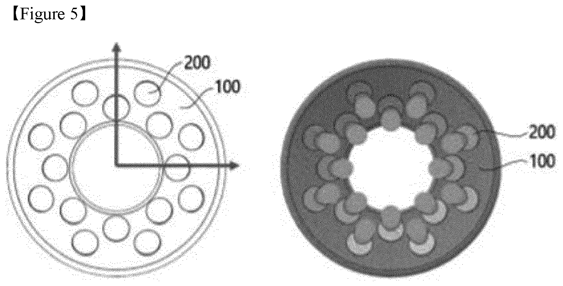

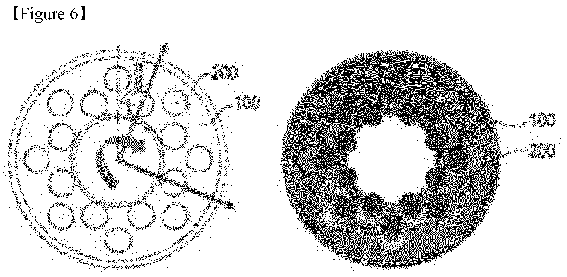

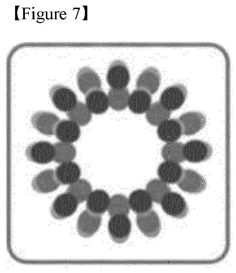

FIG. 4 is a side cross-sectional view illustrating a multi-channel ultrasonic stimulator according to one embodiment of the present invention, and FIGS. 5 to 7 are conceptual diagrams illustrating an application range of ultrasonic waves according to rotation of the housing 100 in one embodiment of the present invention. FIGS. 5 and 6 show a state in which the housing 100 is rotated, and FIG. 7 shows the application range of ultrasonic waves according to the rotation of the housing 100.

Referring to FIG. 4, in one embodiment of the present invention, the multi-channel ultrasonic stimulator further includes a rotator 300 connected to the housing 100 and serving to rotate the housing 100 about a central axis.

The rotator 300 rotates the housing 100 so that the channels 200 arranged in a zigzag pattern in the housing 100 are rotated. This prevents ultrasonic waves from being locally concentrated, thereby uniformly applying ultrasonic waves to a body part of a user. That is, the intensity and application range of the ultrasonic waves are uniformly distributed. Since the channels 200 divided into the first group 210 and the second group 220 are rotated about the center of the housing 100, the application range of ultrasonic waves can be increased, and the number of channels 200 for applying ultrasonic waves to the same area can be reduced.

In addition, the ultrasonic waves emitted from the channels 200 may be incident on a relatively large area rather than a specific point. When the channels 200 emit ultrasonic waves while staying at their respective positions, only a very limited point on a user's body part is stimulated. However, in the present invention, since the positions of the channels 200 change due to the rotation of the housing, a relatively large area of the body part of the user can be stimulated. Accordingly, it is possible to perform uniform ultrasonic stimulation over the entire body of a patent. This reduces locally excessive stimulation concentrated on a specific point on a body part of a user. The therapeutic effect of an anticancer drug can be enhanced through the uniform stimulation of a large area of a body part of a user.

The construction of the rotator 300 will be described in detail below by way of example. For example, the rotator 300 includes a rotary shaft 310 connected to the center of the housing 100 and a rotary motor 320 connected to the rotary shaft 310 and configured to transmit a rotational force to the rotary shaft 310. The rotator 300 further includes a supporting cover 330 provided on the rear surface of the housing 100 to protect the rotary motor 320. The rotary motor 320 is powered by an external power source or its own battery. The supporting cover 330 will be fixedly put on a body part of a user. By stably and fixedly putting the supporting cover 330 on a body part of a user, it is possible to prevent the stimulator from moving when the housing is rotated. The fixedly putting the supporting cover 330 on a body part is achieved by fastening the supporting cover 330 to the body part using an elastic band 400, a string, or the like.

In addition, a bearing (B) is provided between the housing 100 and the supporting cover 330 or between the rotary shaft 310 and the supporting cover 330.

In one embodiment of the present invention, the supporting cover 330 is shaped to surround the outer edge of the housing 100, and the fixing portion 331 described above is provided on one end of the supporting cover 330.

FIG. 4 illustrates a pre-rotation state before the housing 100 is rotated, and FIG. 5 illustrates an application range of ultrasonic waves when the housing 100 is rotated. FIG. 6 shows an application range of ultrasonic waves before the housing 100 is rotated and an application range of ultrasonic waves after the housing 100 is rotated. As illustrated in FIGS. 5 to 7, the rotator 300 intermittently rotates the housing 100 by π/8 (radian) about the center of the housing 100. In another embodiment, the rotation of the housing 100 may be continuous. In one embodiment in which the number of channels 200 is 16, the angle of a channel 200 included in the second group 220 with respect to each adjacent channel 200 included in the first group 210 is π/8 (radian), and the housing 100 is rotated by an angle of π/8 (radian) by the rotator 300. When ultrasonic waves are applied to a body part before and after the housing 100 is rotated, the application ranges of ultrasonic waves emitted from the channels of the first group 210 and the channels of the second group 220 are increased. The operation of the rotary motor 320 is controlled by a controller 500 described below.

The embodiment of the rotator 300 described above can be changed into various structures without departing from the scope of the purpose of rotation of the housing 100, and various configurations may be added or substituted.

In one embodiment of the present invention, a charging unit connected to each of the plurality of channels 200 is additionally included to supply external power to the channels 200. The channels 200 are configured to convert the power supplied through the charging unit into ultrasonic waves and emit the generated ultrasonic waves to the outside. The charging unit is connected to each of the channels 200 and is fixed to the housing 100 to directly supply electric energy to the channels 200. Alternatively, the charging unit may be connected to the supporting cover 330 to supply electric energy to the channels 200 in an indirect manner such as a wireless transfer manner.

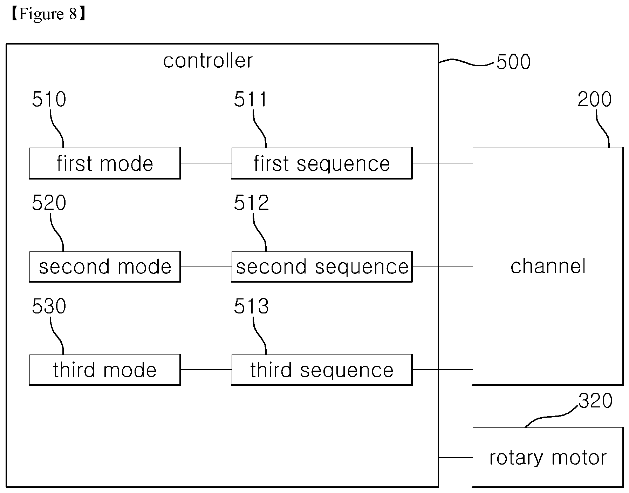

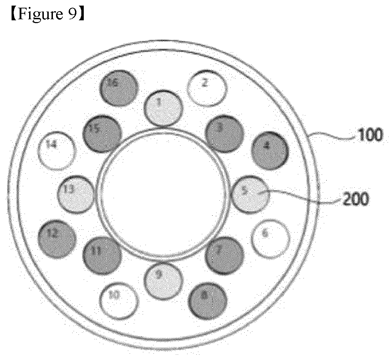

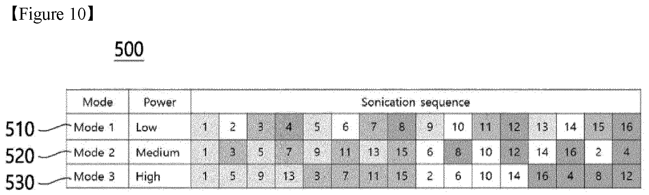

FIG. 8 is a block diagram of a controller 500 used in one embodiment of the present invention, and FIGS. 9 and 10 are conceptual diagrams showing a sequence set by the controller 500. FIG. 9 illustrates a plurality of channels 200 and FIG. 10 illustrates order in which the channels 200 emit ultrasonic waves.

Referring to FIGS. 8 to 10, the present invention includes the controller 500 that adjusts the intensity of ultrasonic waves of the plurality of channels 200. The controller 500 is connected to the channels 200 provided in the housing 100 by cable or wirelessly, and adjusts the intensity of ultrasonic waves applied to a body part by individually controlling the channels 200 according to a sequence that is preset.

The channels 200 that can be independently controlled by the controller 500 may selectively apply ultrasonic waves to a region of a body part. In this case, the intensity of ultrasonic waves emitted from each of the channels 200 can be differently set such that a body part may be locally differently stimulated. Accordingly, it is possible to intensively apply ultrasonic waves to a cancerous portion of a body part of a user and relatively lower the intensity of ultrasonic waves applied to a specific portion of a body part of a user when the specific portion is vulnerable to stimulation.

Hereinafter, the application sequence of ultrasonic waves by the channels 200, which is set by the controller 500 according to the present invention, will be described.

The application sequence set by the controller 500 may be configured such that ultrasonic waves are sequentially emitted from one channel after another along a circumferential direction of the housing 100. When the output value of the ultrasonic waves emitted from each of the channels 200 increases, the spacing of the channels 200 for sequentially emitting ultrasonic waves is increased.

Specifically, the controller 500 can change the application order of the channels 200 according to the predetermined sequence. Hereinafter, for convenience of description, it is assumed that the number of channels 200 is 16.

It is assumed that channels 200 numbered from 1 to 16 are arranged in a clockwise direction in the housing 100. The plurality of channels 200 is divided into a first group 210 composed of odd numbered channels 200 and a second group 220 composed of even numbered channels 200. The channels 200 of the second group 220 and the channels 200 of the first group 210 are alternately arranged. Each of the channels 200 of the first group 210 and a corresponding one of the channels 200 of the second group 220 is spaced by an angle of π/8 (radian). When it is assumed that the housing 100 is rotated by an angle of π/8 (radian), as illustrated in FIG. 4, the channels 200 are positioned between the channels 200 spaced from each other and arranged in a zigzag pattern. The rotation angle may vary depending on the condition, purpose, or environment of application of ultrasonic waves. For example, the rotation angle may be set π/8 (radian) or π/16 (radian).

The controller 500 has three control modes according to the intensity of ultrasonic waves: first mode 510, second mode 520, and third mode 530. The first mode 510 refers to a case in which the output intensity of ultrasonic waves is weak, the second mode 520 refers to a case in which the output intensity of ultrasonic waves is intermediate, and the third mode 530 refers to a case where the output intensity of ultrasonic waves is strong.

In the first mode 510, the channels 200 are operated according to according to a first sequence 511. For example, in the first mode, every channel among the channels numbered from 1 to 16 is sequentially operated. In this mode, since the intensity of the ultrasonic waves emitted from each channel, although every channel applies ultrasonic waves to the user's body part, the user may not feel severe vibration or heat through the ultrasonic stimulation in the first mode.

In the second mode 520, the channels 200 are operated according to a second sequence 521. According to the second sequence, every alternate channel 200 is sequentially operated in this order: channels numbered 1, 3, 5, 7, 9, 11, 13, 15, 6, 8, 10, 12, 14, 16, 2, 4, .... In the case of the second sequence 521, as compared with the first sequence 511, the interval of the channels 200 that emit ultrasound waves is increased to reduce the vibration and heat caused by ultrasonic stimulation so that the user's body does not feel so severe stimulation. In more specific, since ultrasonic waves with an intermediate intensity are emitted from a channel that is numbered 1, emission of ultrasonic waves from the adjacent channel (numbered 2) is skipped, and then emission of ultrasonic waves from the next channel (numbered 3) is performed so that the user may not feel excessive stimulation. In the second mode, the interval between the channels actually emitting ultrasonic waves is adjusted according to the intensity of ultrasonic waves emitted from each channel. For example, when the channels 200 are operated according to the second sequence 521 in the second mode 520, the interval between each of the channels 200 emitting ultrasonic waves may be set to an angle within a range of 45° to 90° with respect to the center of the housing 100.

In the third mode 530, the channels 200 are operated according to a third sequence 531. In the case of the third sequence, the channels numbered 1, 5, 9, 13, 3, 7, 11, 15, 2, 6, 10, 14, 16, 4, 8, 12, ... are sequentially operated one after another. In the third mode, the intensity of ultrasonic waves emitted from each channel is relatively strong. Therefore, the interval between the channels 200 emitting ultrasonic waves is more increased as compared with the second sequence. In the third mode 530 in which the channels are operated according to the third sequence 531, the interval between the channels emitting ultrasonic waves may be set to an angle within a range of from 90° to 180°.

As described above, the controller 500 configured to vary the interval of the operated channels 200 according to the intensity of ultrasonic waves emitted from each channel reduces the stimulation that the user's body part feels and allows uniform ultrasonic stimulation throughout the body part, thereby enabling the stimulated body part to effectively absorb drugs. Since the sequences of the first mode 510 to the third mode 530 are only exemplary, the number of operated channels 200 and the operation order of the channels 200 are determined depending on the purpose of the use of the present invention.

### [Explanation of Reference Numerals]

- 100:: housing
- 200:: channel
- 210:: first group
- 220:: second group
- 300:: rotator
- 310:: rotary shaft
- 320:: motor
- 330:: supporting cover
- 331:: fixing portion
- 400:: elastic band
- 500:: controller
- 510:: first mode
- 511:: first sequence
- 520:: second mode
- 521:: second sequence
- 530:: third mode
- 531:: third sequence
- D1:: first distance
- D2:: second distance
- B:: bearing

### Industrial Applicability

According to the present invention, when performing treatment involving ultrasonic stimulation of a body part, it is possible to uniformly stimulate the body part while preventing excessive stimulation to the body part by controlling the order of channels applying ultrasonic waves and the intensity of ultrasonic waves applied by each channel. In addition, since extensive retrofitting of equipment is not required to implement the present invention, the present invention can be conveniently applied and used.

## Claims

1. A multi-channel ultrasonic stimulator:
a housing with a concavely curved inner surface; and
a plurality of channels provided on the inner surfaced of the housing, spaced from each other at predetermined intervals, and configured to emit ultrasonic waves independently of each other to form an ultrasonic wave field,
wherein the channels are arranged on the inner surface of the housing in a zigzag pattern.

2. The multi-channel ultrasonic stimulator according to claim 1, wherein the channels are divided into a first group distanced from the center of the housing by a first distance and a second group distanced from the center of the housing by a second distance,
the channels in each of the first and second group are arranged at regular intervals,
the second distance is longer than the first distance, and
each of the channels in the second group is spaced from each of the adjacent channels in the first group.

3. The multi-channel ultrasonic stimulator according to claim 2, further comprising a charging unit connected to each of the channels to transfer electric power that is externally input to the channels,
wherein each of the channels converts the received electric power into ultrasonic waves and emits the ultrasonic waves.

4. The multi-channel ultrasonic stimulator according to claim 1, further comprising a rotator connected to the first housing and configured to rotate the housing about a central axis.

5. The multi-channel ultrasonic stimulator according to claim 1, further comprising a controller for adjusting the intensity of ultrasonic waves emitted from the channels,
wherein the controller adjusts the intensity of the ultrasonic waves by controlling each of the channels according to a predetermined sequence.

6. The multi-channel ultrasonic stimulator according to claim 5, wherein the predetermined sequence is set such that the channels sequentially emit the ultrasonic waves in order of the channels arranged in a circumferential direction, and
an interval of the channels that emit the ultrasonic waves increases with an increase in an output value of the ultrasonic wave emitted from the channels.
